(19) Europäisches Patentamt
European Patent Office
Office européen des brevets

(11) **EP 1 839 574 A1**

(12) **EUROPEAN PATENT APPLICATION**

(43) Date of publication:
**03.10.2007 Bulletin 2007/40**

(51) Int Cl.:
***A61B 5/053*** *(2006.01)*

(21) Application number: **06461006.6**

(22) Date of filing: **31.03.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicants:
- **Sakowsky, Jon**
  **Monkton MD 21111 (US)**
- **Wosinski, Stanislaw Adrian**
  **80-280 Gdansk (PL)**

(72) Inventors:
- **Sakowsky, Jon**
  **Monkton MD 21111 (US)**
- **Wosinski, Stanislaw Adrian**
  **80-280 Gdansk (PL)**

(74) Representative: **Rumpel, Alicja**
  **Al. Smiglego-Rydza 29/11**
  **93-281 Lodz (PL)**

(54) **Human organism examination band and human organism examination circuit**

(57)     The band to measure parameters of the human body, provided with the electrodes (5,6 and 7) arranged in such a way that they correspond with the location of meridians of the human wrist and characterized in that it has a form of a disc (1) to wrap the wrist made of the porous silicone, connected through the conductor (2) to the counting instrument (4) (ensuring digital data processing).

**Fig. 1**

EP 1 839 574 A1

Fig. 3

Fig. 7

**Description**

The subject-matter of the invention is the measuring band provided with electrodes, which is connected with the digital device processing the data with the application of appropriate PC software. The measuring band measures parameters of a human body.

The methods to measure parameters of the human body by measuring its resistance ,which have been known and used until nowadays, have been based on studying resistance capacity of the human body in its specific points called meridians. Their arrangement and methods of their finding were determined by the traditional Japanese methods of natural therapy - acupuncture. Moreover, the known methods were not connected with a diagnosis based on measurement of resistance and conductivity of the human body, instead - they were using measurements only in order to find characteristic points - meridians, where electrodes or acupuncture needles should be positioned. On the other hand, precise measurements of resistance were burdened with significant errors resulting from a low repeatability of a correct finding of meridian points on the human body as well as from imperfection of the same methods as based on application of two electrodes - the passive and the active one.

According to the patent description US 4 408 617 the apparatus has been described, which consists of the longitudinal assembly of electrodes and instruments being held by the examined person and informing about finding the point of good electrical conductivity, as well as of the power supply device connected with the electrodes by means of conductors. During the examination, the examined person when holding the apparatus in one hand touches the electrode; at the same time - such a person is isolated from the other electrode placed on a free ending of the apparatus which is not equipped with conductors. After finding the point - meridian, it is possible to send a stimulating impulse to it. This happens on switching the apparatus to the appropriate operating mode.

Next, according to the patent description US 6 306 160 the device has been described, which construction consists of the laser meter to measure resistance of human skin. The main task of the above device is to detect the point with enlarged capability to electrical conductivity and then, to stimulate it with a laser light. Respectively, surface of the skin above the detected point is then heated and consequently - conductivity features of such point are changed.

The other solution envisages apparatuses enabling to detect and stimulate points of meridians much more precisely. According to the patent description US 5 195 517 the devise has been described, which consists of two connected parts. One of them is a board, on which messages are being displayed whereas the second contains elements to operate the device. Additionally, several electrodes are attached to the device. They contain the elements to conduct electric current, which are in contact with the patient's skin during the entire examination, and conductors provided with endings in a form of needles for acupuncture. After introduction of the setting for a given point, the arrangement of meridians will be displayed on the screen and then the needles will be driven into points corresponding with points on the patient's body as displayed on the screen; then stimulation of channels conducting electric current through the patient's body would be started.

The other devise of the similar type but not requiring any knowledge of settings and arrangement of specific points, is the device according to the patent description US 5 546 954. The examined person puts on his/her wrist the band having a form of a clip and containing in its structure a permanent electrode during the entire examination. By means of the conductor, the band is connected with the master with in-built resistance indicator and loudspeaker. Also the electrode ended with the needle is connected to the above master. The current intensity is adjusted with a pedal (foot control) connected to the device. During the examination points of meridians are detected in turn, then the needle is driven accordingly and the stimulation is started just like in previous cases.

The principle of operating the device according to the patent description US 6 285 905 is similar. But in this case the needle for acupuncture was replaced with a thin, conductive slider and a permanent electrode has a form of a suction cap.

All presented solutions require the prior estimation of health of the person to be examined because only after that it is possible to stimulate specific points. Moreover, a necessity to drive needles in a specific way excludes any possibility to operate such devices by inexperienced and unqualified users. Also, this cannot be guaranteed that within the second and following trials a given meridian will be hit; the same point will be chosen once again, especially when several points are situated close one to each other. Similarly, measurements of resistance and conductivity helps only to locate meridians and thus it is not precise and cannot be used to diagnose the patient in detail. Awareness of the fact which points show incorrect conductivity values makes it possible to determine, which organ connected with a given meridian shows malfunction and introduce a respective treatment under the supervision of the professional using electric current and traditional needles for acupuncture. In the case of a healthy organism it does not matter, on which side of the body the respective measurements will be taken, because points responsible for internal organs are situated symmetrically and show equal or quite equal values of current conductivity, but comparison of readings allows to make a detailed diagnosis for the suffering person.

The measuring band provided with electrodes in accordance with the invention is made in a form of a disc wrapped around the wrist, preferably in a form of a clipped belt made of porous silicone. Preferable parameters of the porous silicone to be used in the said invention are presented in the below table:

| Density | BS 4443 | Kg/cm3 | 300(+/-100) |
|---|---|---|---|
| Stress resistance | BS 903 part 2 | Mpa | 1.3 |
| Elongation to the breaking point: | BS 903 part A2 15% max | % | Typical value 207 |
| Compression test (load 40%) | BS 4443 part 1 Method 5B | / | 20 |
| Deformation test by means of compression | BS EN ISO 1856'A' 1857 | % | Max. 15 |
| Range of operating temperatures | From - 60°C up to 200°C (continuous operation) + 250 °C (short- duration operation) | | |

The belt forming the band in connected by means of the conductor with the counting instrument for digital data processing, which can be additionally connected to the personal computer (PC). To the same counting instrument, the second, identical band is attached. Both of them are put in the area of meridians situated on both wrists. On each band, eight electrodes are placed circumferentially: six of them are measuring electrodes, one is the passive electrode and one is the calibrating electrode. Each electrode is built in such a way that material from the inside of the band having a contact with the skin of the examined patient, is impregnated with a solution conducting electric current. This material is introduced into the ring, which inside part is cone-shaped; this ring constitutes an integral part of the band. The other solution consists in such construction, that this part of the electrode which has a contact with the patient's body, is ended with a rubber element conducting electric current or with a conductive metal element e.g. made of silver. The passive and calibrating electrodes (which then functions as the passive one) are situated not far from each other - a couple of millimeters and, at the same time, they are joined together by means of a porous material or any other soft material with conducting properties - impregnated most often with physiological saline, preferably with an addition of lactic acid.

The system in accordance with the invention includes the power supply unit consisting of batteries as the source of electric current and two sub-units : one generating 5V current to feed the control unit and the other - generating 12 V current to feed the operating unit, where the bands are connected to. The operating unit is also composed of two sub-units: the setting sub-unit containing the tuning circuit connected with the electronic key and the measuring sub-unit containing the analog -to-digital converter and the amplifier. The measuring sub-unit is connected to the control unit, which includes the processor and auxiliary circuits such as timer and memory circuits. To the control unit the following are also connected: the user's interface with a display, preferably the LCD one and the keyboard as well as the PC interface, preferably provided with the USB port.

The current of 12V voltage generated by the power supply unit feeds the operating unit and, after being processed in the setting sub-unit with the application of the tuning circuit and the electronic key, it is passed to the measuring bands. The signals received in the operating unit through the intermediary of the bands, are directed through the amplifier and the converter of the measuring sub-unit as the input data to the microprocessor of the control unit. The output data are presented on the display in the user's interface and/or passed to any computer through the computer's interface. The band is made of the porous silicone, characterized by a uniform rate of deformation along the whole length of objects made of this material. Before the main cycle is started, the measurement of current conductivity is taken between the passive and the calibrating electrode within the polyurethane foam impregnated with the conductive liquid. Depending on the resistance value of electrolyte contained in the impregnated material, the automatic determination of the resistor installed in the device is performed. After calibration, the measurements are taken first in the area of left hand meridians whereas on the right hand the assembly of the passive electrode is switched on; next - the same measurement is taken for the other wrist. The processed data are presented in a form of numerical data or graphical data after their transmission through the USB junction into the PC memory. Respectively, the single cycle includes calibration of the apparatus (in order to balance the effect of electrodes drying), and then 12 measurements are taken for individual points i.e. 6 pairs of measurements for corresponding meridians of the left and right hand:

- switching the apparatus to the appropriate electrode in the appropriate band,
- repeated measurements of flowing current intensity taken within 3 seconds,
- storage of the average from the above values.

The complete analysis of human body parameters includes:

- preliminary test consisting of one measuring cycle,
- displaying of results of the preliminary test on the display,
- waiting for the operator's response (the operator starts the second test manually),
- completion of n-cycles of the basic test (the number of measurements - n and time intervals are settled by the operator before the examination),
- for every pair of measurements, respective values are calculated as: a half of the difference between the measured values (d) and the average from the measured values (av.),
- every pair of values is estimated in accordance with the formula: o = d/av. * 100%,
- the total estimation of the measurement is considered as the sum of estimations made in relation to individual pairs; the lower estimation means the better result (the more balanced are the measurements, this is the better).

After the measurements have been taken, their results are presented on the display of the counting instrument and prepared by the microprocessor for their presentation on the PC screen (the PC is connected through the USB junction). The data prepared in this way, after their transmitting to the computer and storing on its hard disk, are presented in a graphical form according to the below algorithm:

- for each measured value, a column is drawn (totally 12 columns),
- columns are grouped in pairs - for the same point on the right and left hand (totally 6 pairs),
- the column height depends on the measured value, but not linearly. The higher is the value, the more concentrated is the scale (this means for example that difference in heights of columns for values 10 and 20 is larger than in the case of values 100 and 110 - this is connected with the method of measurements estimation as described below.

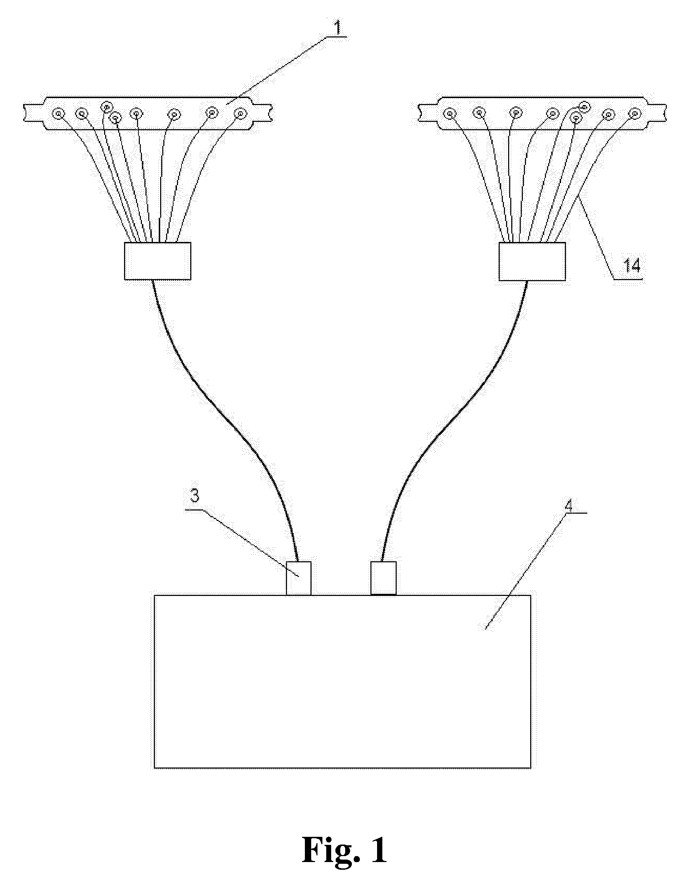

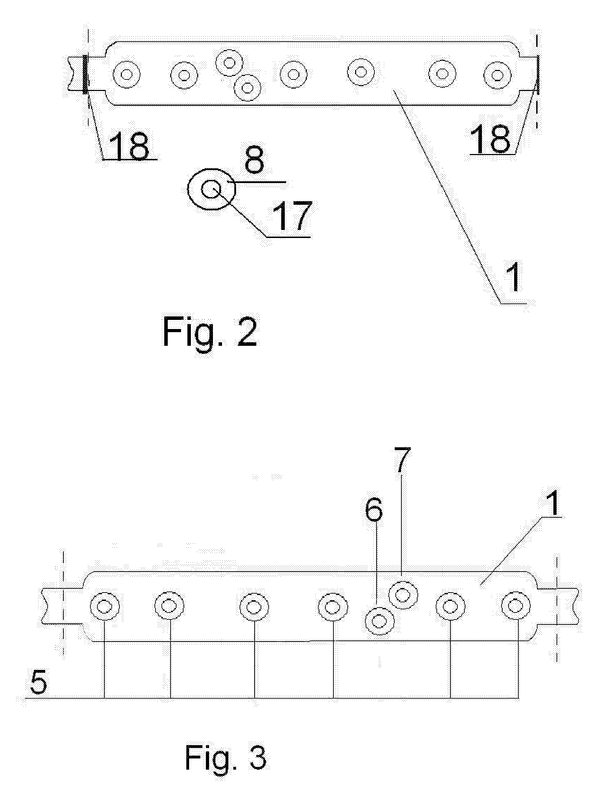

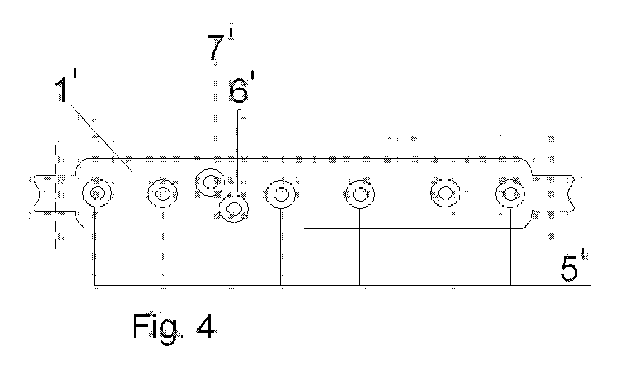

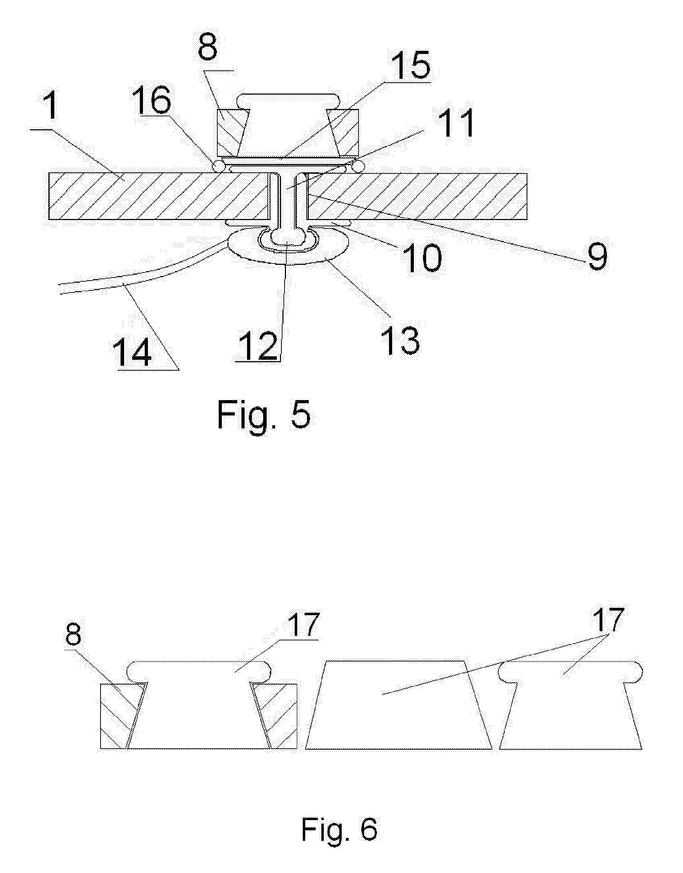

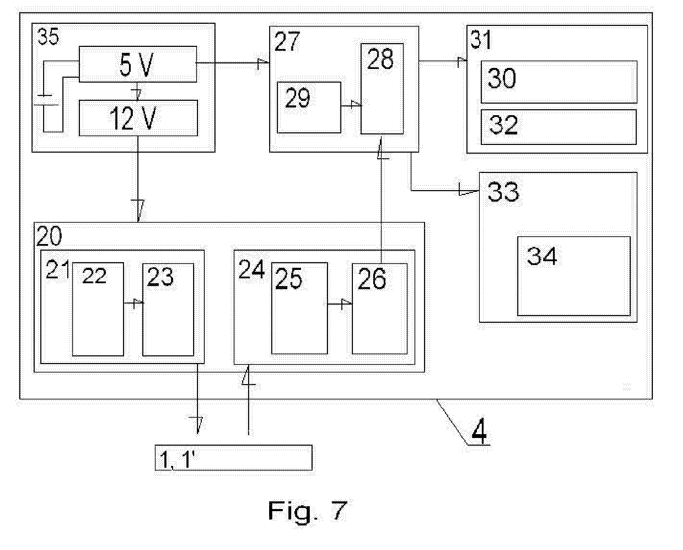

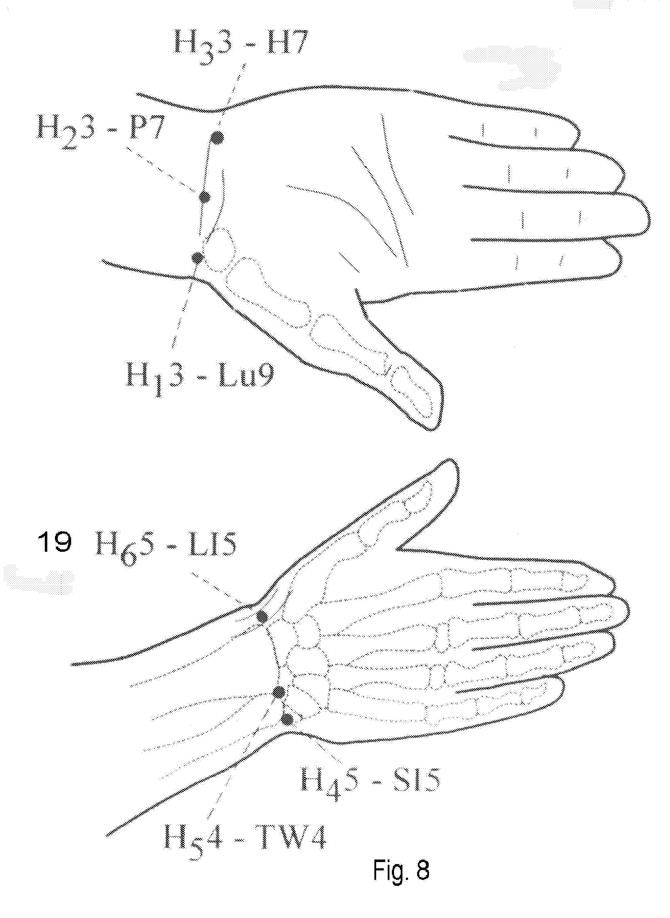

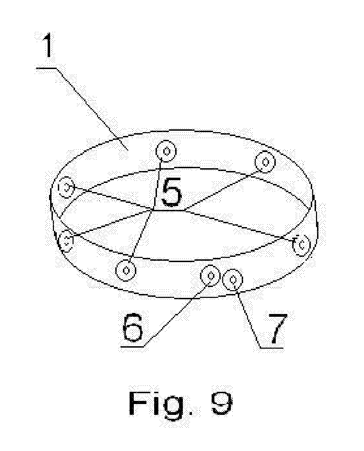

The measuring band provided with electrodes according to the invention is shown in the figures where the Fig. 1 reflects the diagrammatic lay-out of the bands and the counting instrument. Fig. 2 presents the arrangement of electrodes on the measuring bands, Fig. 3 presents the arrangement of electrodes on the right band, Fig. 4 presents arrangement of electrodes on the left band. In the Fig. 5 the structure of electrodes is shown, and in the Fig. 6 the detail of polyurethane ring with a recess is presented. The operating algorithm for the counting instrument is presented in the Fig.7. The measurement points on both wrists are shown in the Fig. 8.

The measuring bands provided with the electrodes 1 are made of the porous silicone and connected by means of the system of the main conductors 2, which are ended with the input plug 3 situated on the one side of the counting instrument and with the outputs leading to successive measuring electrodes situated on the other side of the above counting instrument 4. The structure of the measuring band 1 includes the measuring electrodes 5 , one passive electrode 6 and one calibrating electrode 7. The passive electrode 6 and the calibrating electrode 7 are situated at a distance of several millimeters one from the other and surrounded with the conductive, porous silicone impregnated with the physiological saline and having a form of the ring with a recess 8. The other electrodes have the similar structure. Into the hole 9 made in the material of the band, two parts of the rivet are inserted - the part 10 and the other part 11 entering the first one. They are clipped in such a way, that on the outer part of the band the snap connection 12 is created, which is provided with a catch 13 with the outgoing conductor 14, which is connected further to the main conductor 2. To the opposite surface of the rivet - i.e. to its part 11 - the silver sequin is soldered. Between the material of the band 1 and the soldered sequin 15 there is a layer of a silicone glue 16 stabilizing the above joint. The ring 8 with an inside recess having a form of a truncated cone (with a cross-section of an isoscales trapezoid) is fitted to the outer surface of the silver sequin 15 in such a way, that its longer base is in contact with the silver sequin 15. In the said recess, the inset 15 having a form of a truncated cone and made of the foam impregnated with the conductive substance, is placed. The inset shape corresponds with dimensions of the recess in the ring 8, but it slightly protrudes above the outer edge of the recessed ring 8. The band is made in a form of an elastic belt, which is clipped with the clip 18 on the wrist of the examined person. Then, the insets 17, protruding from the recessed rings, will be deformed but this deformation will not have any influence on the pressure acting on the examined person skin.

Prior to wearing the bands 1, the insets 17 of the recessed rings 8 are impregnated with any substance to conduct electric current i.e. physiological saline and only then they are wrapped around the wrist of the examined person and clipped with the clip 18. After placing the bands 1 on the patient's wrists in such a way that the first one out of terminal electrodes is situated in the point 19 ($H_6$5- Li5) according to the Ryodoraku's terminology, the endings of the main conductors 2 finished with the plugs 3 are connected to the correct sockets of the counting instrument 4. Next, the counting instrument 4 is energized and started accordingly; after that the calibration and automatic setting of the resistor of the counting instrument 4 is accomplished. The measuring cycle is carried out automatically and its result is displayed on the display of the counting instrument 4 and it is a comparison of electric conductivity of the human body observed in both directions - right hand-to- left hand and left hand-to-right hand.

The data referring to the resistance and conductivity of the human body received through the measuring bands 1, are transmitted to the operating unit 20 of the counting instrument 4. The reading is made on the ground of the measurement of differences in the flow of electric current sent from the setting sub-unit what is performed by means of the tuning circuit 22 and electronic key 23 of the setting sub-unit and the measuring sub-unit 24, which includes the signal amplifier 25 and analog-to-digital converter 26.

The processing and presentation of the data is based on the algorithm but it can be also formulated as the algorithm of the complete measuring cycle or as the algorithm of separate, single measurements.

The algorithm of the complete measuring cycle can be determined in the following way:

- preliminary test consisting of one measuring cycle,
- displaying of results of the preliminary test on the display,
- waiting for the operator's response (the operator starts the second test manually),
- completion of n-cycles of the basic test (the number of measurements - n and time intervals are settled by the operator before the examination),
- for every pair of measurements, respective values are calculated as: a half of the difference between the measured values (d) and the average from the measured values (av.),
- every pair of values is estimated in accordance with the formula:

$$o = d/av. * 100\%,$$

- the total estimation of the measurement is considered as the sum of estimations made in relation to individual pairs; the lower estimation means the better result (the more balanced are the measurements, this is the better).

On the other hand, the algorithm of the separate measuring cycle is determined by the below mentioned procedure:

- calibration of the apparatus (in order to balance the effect of electrodes drying),
- realization of the complete measuring cycle consisting in 12 measurements taken in respective measurement points,
- switching the apparatus to the appropriate electrode in the appropriate band,
- repeated measurement of flowing current intensity taken within 3 seconds,
- storage of the average from the above values.

After taking measurements, their results are presented on the display of the counting instrument and prepared by the microprocessor for presentation on the screen of the PC computer connected through the USB junction. The data, as prepared in the above mentioned way after their transmitting to the computer and storing on its hard disk, are then presented in a graphical form according to the below algorithm:

- for each measured value, a column is drawn (totally 12 columns),
- columns are grouped in pairs - for the same point on the right and left hand (totally 6 pairs),
- the column height depends on the measured value, but not linearly. The higher is the value, the more concentrated is the scale (this means for example that difference in heights of columns for values 10 and 20 is larger than in the case of values 100 and 110 - this is connected with the method of measurements estimation as described below.

The obtained data are next processed in the control unit 27 by the microprocessor 28 coupled with the auxiliary sub-units 29. After the information has been processed, the device presents the information on correctness of the read result and measured values in a form of the message displayed on the LCD 30 display being a part of the user's interface. The user's interface 31 is equipped also with a keyboard 32 to operate the device. Despite displaying the results of the examination on the LCD 30 display, there is also a possibility to present them on the screen of the PC 33 provided with the sub-unit of data transmission USB 34.

Before the presentation of the results, from among all records referring respective cycles we chose the most reliable one complying with the below programming criteria:

- for every pair of measurements, respective values are calculated as: a half of the difference between the measured values (d) and the average from the measured values (av.),
- every pair of values is estimated in accordance with the formula:

$$o = d/av. * 100\%,$$

- the total estimation of the measurement is considered as the sum of estimations made in relation to individual pairs; the lower estimation means the better result (the more balanced are the measurements, this is the better).

The devise is energized by the power unit 35, which is fed from the batteries and incorporated sub-units generating 5 V and 12 V voltages, which are then transmitted to the control unit 27 and the operating unit 20.

The subject-matter of the invention has been made of materials meeting all sanitary, hygienic and safety requirements. The automatic mode of operation allows to take measurements also by persons who are not used to apply such measuring apparatuses and who are not familiar with measurement methods based on traditional acupuncture and the Ryodoraku's method.

Interpretation of a symmetry of such input data in a form of a numerical statement, preferably a diagram, makes it possible not only to diagnose health condition of the examined person but also to come to a conclusion on various factors having an influence on the patient's condition when repeating examinations under different conditions and after applying a different stimulus. Taking into consideration the objectiveness of the examinations, it is preferable that control, comparative examinations are conducted under the same conditions and with patients of the similar psychophysical condition. On the ground of studies referring to the classic methods of Ryodoraku's conductivity measurements, which constitute the basis for the method according to this invention, it was proved that such factors as sleep length and stress influenced the human body capability to conduct electric current. During the examination conducted with the application of the band according to the invention, the bands remained clipped all the time on the patient and were not taken off. That is why the repeatability and effectiveness of measurements of meridians conductivity during the second and every successive trial is close to 100%. Moreover, properties of the porous silicone, from which the band is made, ensures that the pressure acting on the examined point is equal and appropriate.

Marks on figures

1.   Measuring band made of porous silicon
2.   Main conductor
3.   Input plug
4.   Counting instrument
5.   Measuring electrodes
6.   Passive electrode
7.   Calibrating electrode
8.   Polyurethane ring with a recess
9.   Rivet hole
10.  External part of rivet
11.  Internal part of rivet
12.  Snap connection
13.  Catch
14.  Electrode conductor
15.  Silver sequin
16.  Silicone glue
17.  Polyurethane insets of the ring with a recess
18.  Clip
19.  Point H65- Li5
20.  Operating unit
21.  Regulating circuit
22.  Tuning circuit
23.  Electronic key
24.  Measuring unit
25.  Amplifier
26.  Analog-to-digital converter
27.  Control unit
28.  Microprocessor
29.  Auxiliary sub-units
30.  LCD display
31.  User's interface
32.  Keyboard
33.  PC interface
34.  USB data transmission sub-unit

**Claims**

1.   The band to measure parameters of the human body, provided with the electrodes 5, 6 and 7 arranged in such a way that they correspond with the location of meridians of the human wrist and **characterized in that** it has a form of a disc 1 to wrap the wrist made of the porous silicone, connected through the conductor 2 to the counting instrument 4 (ensuring digital data processing).

2.   The band according to the claim 1 and **characterized in that** the disc has a form of the belt to be clipped.

3.   The band according to the claim 1 or 2 and **characterized in that** the porous silicone of the disc 1 has the following parameters:

| | | | |
|---|---|---|---|
| Density | BS 4443 | Kg/cm3 | 300(+/-100) |
| Stress resistance | BS 903 part 2 | Mpa | 1.3 |
| Elongation to the breaking point: | BS 903 part A2 15% max | % | Typical value 207 |
| Compression test (load 40%) | BS 4443 part 1 Method 5B | / | 20 |
| Deformation test by means of compression | BS EN ISO 1856'A' 1857 | % | Max.15 |
| Range of operating temperatures | From - 60°C up to 200°C (continuous operation) + 250 °C (short- duration operation) | | |

4. The band according to the claim 1 or 2 or 3 and **characterized in that** on the said disc 1 of the band, at least one measuring electrode 5 is placed circumferentially corresponding with the location of at least one meridian on the human wrist.

5. The band according to the claim 1 or 2 or 3 or 4 and **characterized in that** on the said disc 1 of the band, 6 measuring electrodes 5 are placed corresponding with the location of meridians on the human wrist.

6. The band according to the claim 1 or 2 or 3 or 4 and **characterized in that** on the said disc of the band, six measuring electrodes 5 are placed corresponding with the location of meridians on the human wrist as well as one passive electrode 6 and one calibrating electrode 7 is also placed on it.

7. The band according to the claim 1 or 2 or 3 or 4 or 6 and **characterized in that** from the inside of the material 17 of the band having a contact with the skin of the examined patient is impregnated with a solution conducting electric current. This material is introduced into the ring 8, which inside part is cone-shaped; this ring constitutes an integral part of the band.

8. The band according to the claim 1 or 2 or 3 or 4 or 6 and **characterized in that** each electrode 5, 6 and 7 in a part, which has a contact with the patient's body, is ended with a rubber element conducting electric current.

9. The band according to the claim 1 or 2 or 3 or 4 or 6 and **characterized in that** each electrode 5, 6 and 7 in a part, which has a contact with the patient's body, is ended with a conductive metal element.

10. The band according to the claim 1 or 2 or 3 or 4 or 6 or 7 or 8 or 9 and **characterized in that** the passive electrode 6 and the calibrating electrode 7 are situated not far from each other and, at the same time, they are joined together by means of a porous material and/or any other soft material with conducting properties and impregnated with a solution conducting electric current.

11. The system to measure parameters of the human organism, **characterized in that** it contains the power supply unit 35 to feed the control unit 27 and the operating unit 20. The user's interface 31 is attached to the control unit whereas the electrodes 5, 6 and 7 of the measuring bands for the right and left hand are attached to the operating unit 20.

12. The system according to the claim 1 and **characterized in that** the operating unit 20 is composed of two sub-units: the setting unit 21 containing the tuning circuit 22 connected with the electronic key 23 and the measuring sub-unit 24 containing the analog-to-digital converter 26 and the amplifier 25.

13. The system according to the claim 1 and **characterized in that** the control unit 27 includes the processor 28 and auxiliary circuits such as timer and memory circuits.

14. The system according to the claim 1 and **characterized in that** PC user's interface 31 is connected to the control unit 27.

15. The system according to the claim 1 and **characterized in that** the PC interface 33 is connected with the control unit 27 by means of the USB 34 port.

**Fig. 1**

18　　　　　　　　　　　　　18

8

17

1

Fig. 2

7

6　　　　　　　　1

5

Fig. 3

Fig. 4

Fig. 5

Fig. 6

Fig. 7

$H_3 3 - H7$

$H_2 3 - P7$

$H_1 3 - Lu9$

19 $H_6 5 - LI5$

$H_4 5 - SI5$

$H_5 4 - TW4$

Fig. 8

Fig. 9

# EP 1 839 574 A1

**European Patent Office**

## EUROPEAN SEARCH REPORT

Application Number

EP 06 46 1006

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 971 366 A (MOTOYAMA HIROSHI) 27 July 1976 (1976-07-27) | 11-15 | INV. A61B5/053 |
| Y | * column 5, line 9 - column 6, line 60 * | 1-10 | |
| A | ORJAN G MARTINSEN* O G ET AL: "Line Patterns in the Mosaic Electrical Properties of Human Skin-A Cross-Correlation Study" IEEE TRANSACTIONS ON BIOMEDICAL ENGINEERING, IEEE SERVICE CENTER, PISCATAWAY, NJ, US, vol. 48, no. 6, June 2001 (2001-06), XP011007095 ISSN: 0018-9294 page 731, section 'Methods' figure 3 | 11-15 | |
| Y | DE 102 26 839 A1 (XU TENGJIAO [DE]; XU TIE [DE]; ZHANG YANMING [DE]) 15 January 2004 (2004-01-15) * paragraphs [0007] - [0009] * | 1-10 | |
| Y | US 2002/191011 A1 (RASOULI FIROOZ [US]) 19 December 2002 (2002-12-19) * paragraph [0033] * | 1-10 | TECHNICAL FIELDS SEARCHED (IPC) A61B A61H A61N |
| A | GB 2 367 621 A (SEVEN OF NINE LTD [GB]) 10 April 2002 (2002-04-10) * page 5, line 2 - line 3 * * page 7, line 1 - line 3 * * page 7, line 12 - page 8, line 5 * | 1,11 | |

The present search report has been drawn up for all claims

| Place of search | Date of completion of the search | Examiner |
|---|---|---|
| The Hague | 10 October 2006 | Knüpling, Moritz |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 06 46 1006

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2006

| Patent document cited in search report | | Publication date | Patent family member(s) | Publication date |
|---|---|---|---|---|
| US 3971366 | A | 27-07-1976 | NONE | |
| DE 10226839 | A1 | 15-01-2004 | NONE | |
| US 2002191011 | A1 | 19-12-2002 | NONE | |
| GB 2367621 | A | 10-04-2002 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4408617 A
- US 6306160 B
- US 5195517 A
- US 5546954 A
- US 6285905 B